# EUROPEAN PATENT SPECIFICATION

(11) **EP 1 913 885 B1**
(45) Date of publication and mention of the grant of the patent: **30.09.2009**
(21) Application number: 07020160.3
(22) Date of filing: 15.10.2007
(51) Int. Cl.: A61B 17/70

(54) **Vertebra connection member**
Wirbelverbindungsglied
Membre de connexion vertébrale

(30) Priority: 20.10.2006 US 584216
(43) Date of publication of application: 23.04.2008
(73) Proprietor: Showa Ika Kohgyo Co., Ltd., Nagoya-shi Aichi 465-0024 (JP)
(72) Inventor: Oribe, Kazuya, Minato-ku Tokyo 105-0011 (JP); Takamido, Hiroshi, Nagoya-shi Aichi 465-0024 (JP)
(74) Representative: Schmidt, Karsten

(56) References cited:
- EP-A- 1 402 828
- WO-A-20/06089292
- US-A- 5 628 740
- US-A- 6 077 262
- US-A1- 2005 277 928
- US-A1- 2006 111 715

## Description

### BACKGROUND OF THE INVENTION

### Field of the Invention

The present invention relates to a vertebra connection member that connects a plurality of vertebrae via a rod.

### Description of the Related Art

A vertebra connection member described, for example, in Japanese Patent Application Laid-Open No. 2004-097705 is known as a member for connecting a plurality of vertebrae via a rod. In the vertebra connection member, a spherical head section is provided at one end of a screw member to be screwed into a vertebra, this head section is received by a connection member that connects a rod and the screw member, and the screw member is held so that it can be tilted with respect to the connection member. As a result, the connection member can be positioned at a desired angle, and a plurality of vertebrae tilted at different angles can be connected via the rod.

EP-A2-1402828 discloses a vertebra connection member according to the preamble of the independent claims. The connection members disclosed in this piece of prior art, however, do not show a roughened surface nor a slipping providing agent, and they do not include a head section holding member having a slit provided from the lower end to the upper end.

### SUMMARY OF THE INVENTION

Having analyzed the above-described vertebra connection member, the inventors have discovered that because the head section of the screw member is formed to have a spherical shape, sometimes the head section is not sufficiently fixed to the connection member and a plurality of vertebrae cannot be connected in a desired manner via a rod.

An object of the present invention is to provide a vertebra connection member that can inhibit the displacement of the head section with respect to the connection member.

A vertebra connection member in accordance with the present invention connects a plurality of vertebrae via a rod and comprises a screw member having a spherical head section provided at one end of the screw member and a screw section for being screwed into the vertebra; a connection member into which the screw member is inserted and that connects the screw member and the rod, said connection member having a head support section receiving the head section and supporting the screw member so that the screw member can be tilted, said connection member further having a rod insertion section into which the rod may be inserted; a head section holding member that is inserted between the rod and the head section inside the connection member, said head section holding member having a head holding section for holding the head section and a rod holding section, inserted into the rod insertion section, for holding the rod; and a pushing member that pushes the head section toward the head support section via the rod and head section holding member, wherein at least one of the head section, head support section, and head holding section is subjected to surface roughening treatment.

In the vertebra connection member in accordance with the present invention, by roughening the surface of at least one section of the head section, head support section, and head holding section, the friction resistance of the head section and the head support section and/or head holding section is greatly increased. Therefore, by pushing the head section toward the head support section by the pushing member via the rod and head holding member, the head section can be strongly fixed to the connection member when the screw member and rod are connected. As a result, the displacement of the head section with respect to the connection member can be inhibited and the vertebrae can be reliably connected.

The aforementioned surface roughening treatment is preferably performed by sandblasting. By employing such configuration, the surface roughening treatment can be performed in a simple manner.

Furthermore, the head section holding member has an annular section having the head holding section on the lower inner wall surface and a slit provided from the lower end to the upper end. In such vertebra connection member, if the head section holding member and head section are pushed by the pushing member toward the head support section via the rod, the head section of the screw member is pushed into the annular section. As a result, the head section holding member is elastically deformed so as to expand, and the restoration force of the head section holding member acts to squeeze the head section. Therefore, the head section holding member and head section can be stronger fixed to each other.

The head section holding member is preferably provided with a notch in the lower end portion of the annular section. In such vertebra connection member, if the head section holding member and head section are pushed by the pushing member toward the head support section via the rod, the notch of the head section support member will be impressed into the head section of the screw member. As a result, the head section holding member and head section can be stronger fixed to each other.

The head support section and the head holding section are preferably formed to have a concave curved surface shape that conforms to the head section. In such vertebra connection member, the contact surface area of the head section, head support section, and head holding section is increased, whereby the head section can be stronger fixed to the connection member.

At least one section from among the head section, head support section, and head holding section is provided with a protrusion. In such vertebra connection member, if the head section holding member and head section are pushed by the pushing member toward the head support section via the rod, the protrusion will be impressed into the head section, head support section, and/or head holding section. As a result the head section can be fixed to the connection member with higher reliability.

The pushing member is preferably screwed on the inner side of the connection member. As a result, the external dimensions of the vertebra connection member can be reduced and the load applied to the tissue surrounding the vertebrae connected by the vertebra connection member can be decreased.

A vertebra connection member in accordance with the present invention connects a plurality of vertebrae via a rod and comprises a screw member having a spherical head section provided at one end of the screw member and a screw section for being screwed into the vertebra; a connection member into which the screw member is inserted and that connects the screw member and the rod, said connection member having a head support section receiving the head section and supporting the screw member so that the screw member can be tilted, said connection member further having a rod insertion section into which the rod may be inserted; a head section holding member that is inserted between the rod and the head section inside the connection member, said head section holding member having a head holding section for holding the head section and a rod holding section, inserted into the rod insertion section, for holding the rod; and a pushing member that pushes the head section toward the head support section via the rod and the head section holding member, wherein a slipping preventing agent or an adhesive is applied to at least one of a surface between the head section and the head support section and a surface between the head section and the head holding section.

In the vertebra connection member in accordance with the present invention, a slipping preventing agent or an adhesive is applied to the surface of at least one section from among the head section, head support section, and head holding section, whereby slipping between the head section and the head support section and/or head holding section is inhibited. Therefore, when the screw member and rod are connected by pushing the head section holding member and head section by the pushing member toward the head support section via the rod, the head section can be strongly fixed to the connection member. As a result, the displacement of the head section with respect to the connection member can be inhibited and the vertebrae can be reliably connected.

A fuller understanding of the present invention will be had from the following detailed description and the appended drawings. Those description and drawings are presented merely to illustrate the present invention and should not be construed as limiting.

### BRIEF DESCRIPTION OF THE DRAWINGS

Fig. 1 illustrates the state in which the vertebra connection member in accordance with the present invention is connected to a rod;
Fig. 2 is an exploded side view of the vertebra connection member;
Fig. 3 is a side view showing the screw member;
Fig. 4 is a side view showing the head section of the screw member;
Fig. 5 shows the connection member, (a) is a top view, (b) is a front view, and (c) is a side view;
Fig. 6 is a VI-VI cross section of Fig. 5;
Fig. 7 shows a head section support member, (a) is a perspective view that is viewed from above, (b) is a perspective view that is viewed from below;
Fig. 8 shows part of the vertebra connection member; (a) is a top view; (b) is a side view;
Fig. 9 is a side view showing a state in which the screw member is inclined;
Fig. 10 is a side view showing a vertebra connection member in which a slipping preventing agent or an adhesive is applied to the head section, head support section, and head holding section; and
Fig. 11 is a side view showing a vertebra connection member in which protrusions are provided on the head section, head support section, and head holding section.

### DESCRIPTION OF THE PREFERRED EMBODIMENTS

The preferred embodiments of the present invention will be described below in greater detail with reference to the appended drawings. In the drawings, identical elements are assigned with identical reference symbols to avoid the redundant explanation.

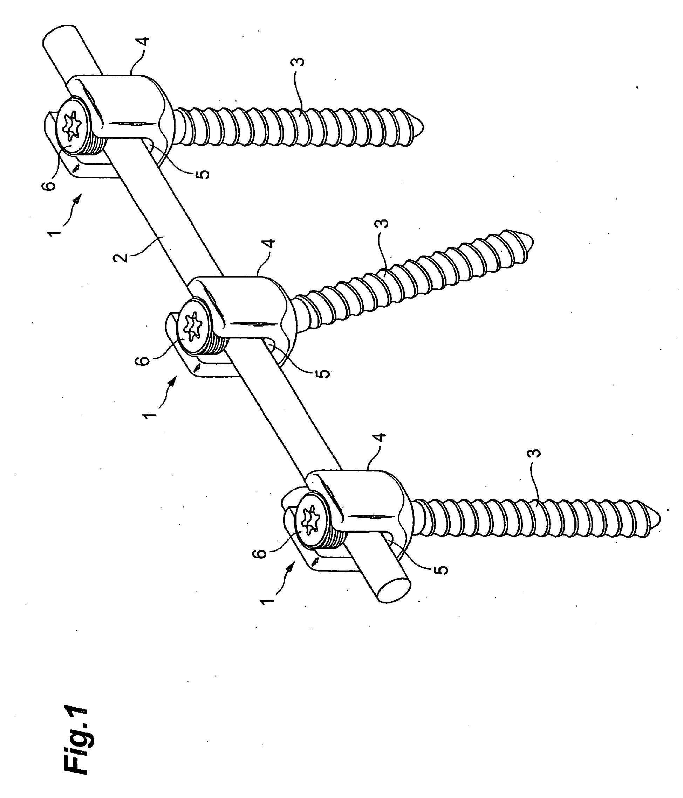

A vertebra connection member 1 in accordance with the present invention serves to connect and fix a plurality of vertebrae. In actual use, as shown in Fig. 1, a plurality of vertebrae are connected and fixed by fixing a plurality of vertebra connection members 1 to one rod 2.

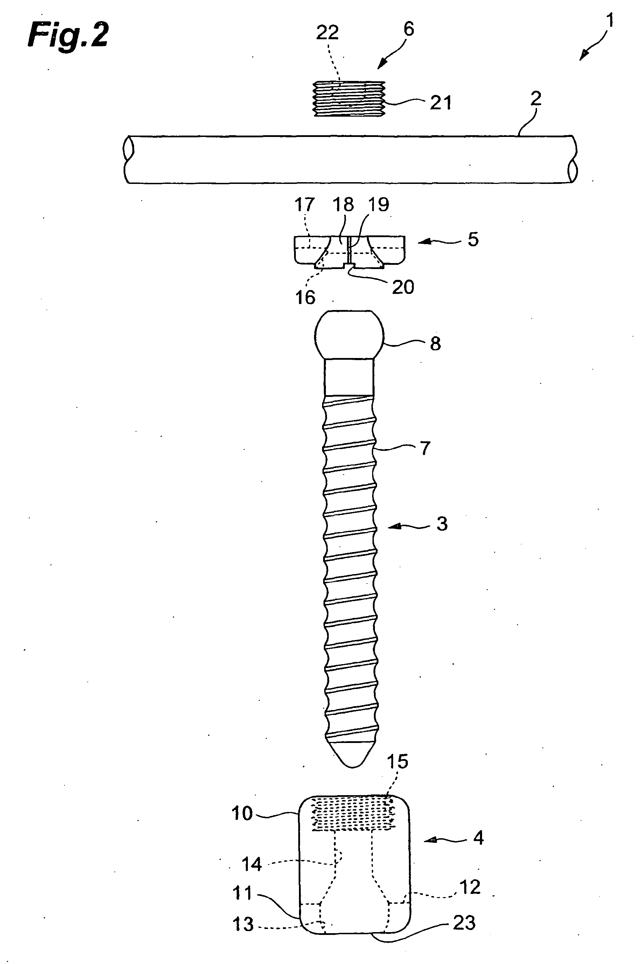

As shown in Fig. 2 the vertebra connection member 1 comprises a screw member 3 for screwing into a vertebra, a connection member 4 for connecting the screw member 3 and the rod 2, a head section holding member 5 that is inserted between the screw member 3 and rod 2 inside the connection member 4, and a pushing member 6 that is screwed into the connection member 4 and pushes the rod 2 and screw member 3 and fixes them to the connection member 4.

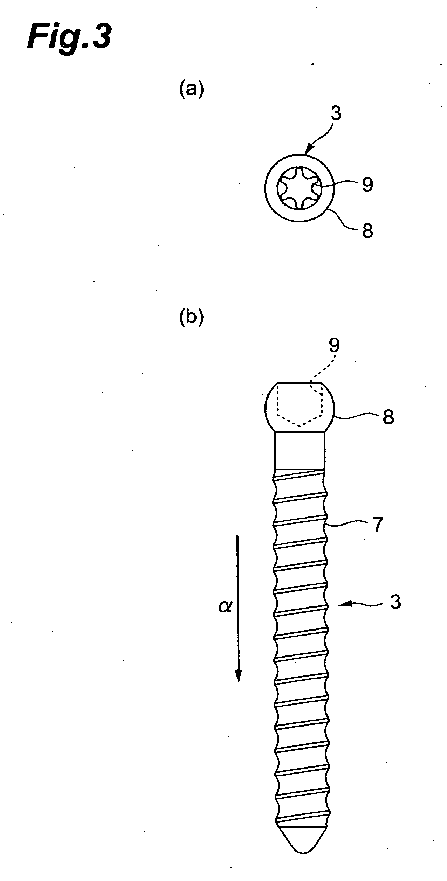

The screw member 3 is formed from a corrosion-resistant material such as titanium and subjected to anodization treatment to form a coating film on the entire surface thereof. The screw member 3, as shown in Fig. 3, is formed of a rod-shaped threaded section 7 provided with a thread for screwing into a vertebra and a substantially spherical head section 8 provided at the rear end in the direction of screwing the threaded section 7 of the screw member 3 into the vertebra (referred to hereinbelow as "screw-in direction α").

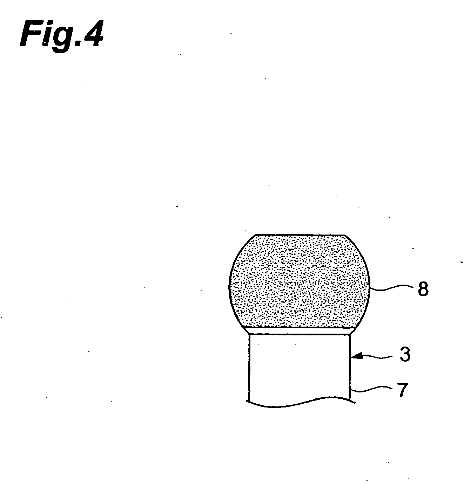

In the head section 8, a star-shaped receded concave section 9 is formed in the screw-in direction α of the screw member 3 so as to insert a star-shaped wrench (not shown in the figure) for rotating the screw member. Further, as shown in Fig. 4, the outer surface of the head section 8 is subjected to sandblasting treatment.

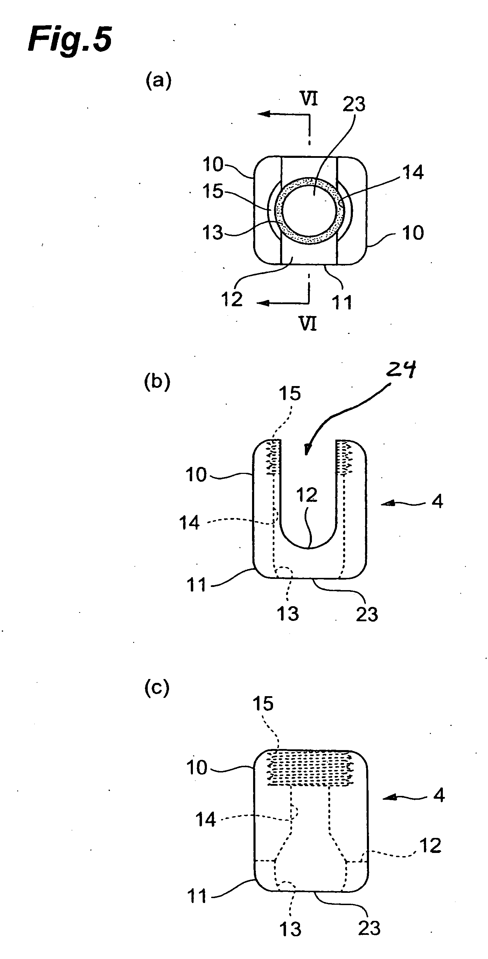

The connection member 4 is formed from a corrosion-resistant material such as titanium and subjected to anodization treatment to form a coating film on the entire surface thereof. As shown in Fig. 5, the connection member 4 is formed to have a U-shaped cross section by a pair of side wall sections 10 that are provided upright as two peaks and a bottom wall section 11 that joins the lower parts of the pair of side wall sections 10.

The distance between a pair of side wall sections 10 is somewhat larger than the maximum diameter of the rod 2 and somewhat smaller than the maximum diameter of the head section 8 of the screw member 3. On the inner side of the pair of side wall section 10, a groove 14 for a head section that extends in the up-down direction (up-down direction in Fig. 5) and passes through the center in the width direction is formed to guide the head section 8 to the lower section. The groove 14 for a head section is formed to have a circular arc surface having an inner diameter somewhat larger than the maximum diameter of the head section 8 so that the head section 8 can be inserted therein. A female threaded section 15 for screwing the pushing member 6 is formed on the inner side of the pair of side wall section 10 above the groove 14 for a head section.

A port 23 for inserting the screw member 3 in the up-down direction is formed in the bottom wall section 11, and a head support section 13 that receives the head section 8 of the screw member 3 and supports the screw member 3 so that the screw member can be tilted is formed in the inner wall surface of the bottom wall section 11 where the port 23 is provided.

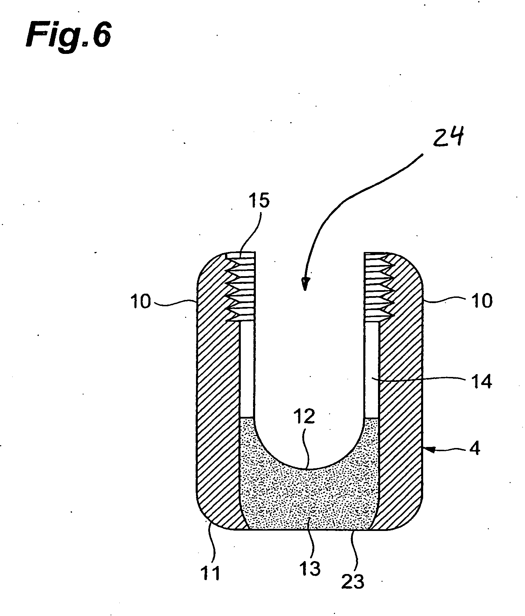

The head support section 13 has a circular inner wall surface of the same diameter as the groove 14 for a head section from the upper part to the vicinity of the lower end, and the lower end section thereof is formed to have a concave curved surface that conforms to the outer shape of the head section 8 so that the lower end has a diameter larger than the outer diameter of the threaded section 7 and smaller than the maximum diameter of the head section 8. As shown in Fig. 6, the inner surface of the head support section 13 that supports the head section 8 so that it can be tilted with respect to the connection member 4 is subjected to sandblasting treatment. The head support section 13 is not limited to the above-described shape and may be of any shape, provided that the threaded section 7 can be inserted therein and the head section 8 can be supported so that it can be tilted with respect to the connection member 4.

Furthermore, a pair of side wall sections 10 form a rod insertion section 24 for inserting the rod 2 in the direction (crossing direction) different from that of the screw member 3. Furthermore, the bottom wall section 11 or a pair of side wall sections 10 and the bottom wall section 11 form a positioning section 12 for positioning the head section holding member 5.

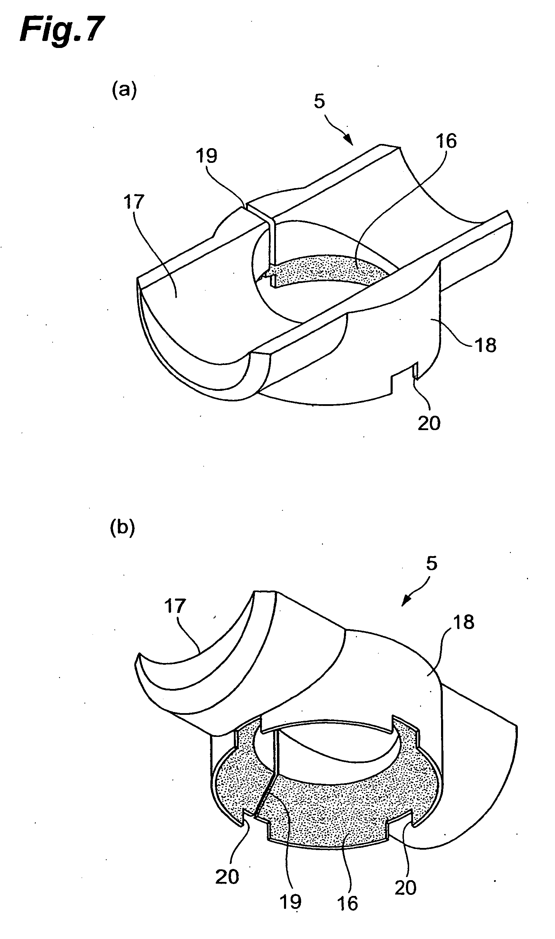

The head section holding member 5 is formed from a corrosion-resistant material such as titanium and subjected to anodization treatment to form a coating film on the entire surface thereof. As shown in Fig. 7, the head section holding member 5 is formed by a tubular cylindrical section 18 provided with a head holding section 16 for holding the head section 8 of the screw member 3 so that the head section can be tilted with respect to the connection member 4 and a rod holding section 17 that extends sidewise from the upper end section of the cylindrical section 18 and serves to hold the rod 2.

The outer shape of the cylindrical section 18 is formed to a size enabling the insertion into the port 23 of the connection member 4. The inner wall surface in the upper section of the cylindrical section 18 is formed as a circle of the same diameter, and the inner diameter thereof is formed to be smaller than the largest diameter of the head section 8 and larger than the largest diameter of the concave section 9 so as to hold the head section 8 and to insert a star-shaped wrench into the concave section 9 formed in the head section 8. Furthermore, a head holding section 16 is formed on the lower inner wall surface of the cylindrical section 18. The inner diameter of the head holding section 16 expands toward the lower end, and the head holding section is formed as the concave curved surface that conforms to the outer shape of the head section 8 in order to hold the head section 8. The inner surface of the head holding section 16 for holding the head section 8 is subjected to sandblasting treatment. A plurality of notches 20 are formed at the lower end section of the cylindrical section 18 where the head holding section 16 is formed. A slit 19 is provided from the lower end upward in the cylindrical section 18. The slit 19 may be formed from the lower end of the cylindrical section 18 upward, but in the present invention the slit 19 is provided from the lower end to the upper end, and the side wall section of the cylindrical section 18 is divided by the slit 19 extending in the up-down direction.

The rod holding section 17 is formed as a half-pipe. The lower surface of the rod holding section 17 is formed to have a convex curved surface shape such that conforms to the positioning section 12 of the connection member 4, and the upper surface of the rod holding section 17 is formed to have a concave curved surface shape such that conforms to the outer shape of the rod 2. Furthermore, the rod holding section 17 extends so as to follow the positioning section 12 in a state where the head holding section 16 is inserted into the port 23 of the connection member 4, and the rod holding section is formed to have almost the same length as the positioning section 12.

The pushing member 6 is formed from a corrosion-resistant material such as titanium and subjected to anodization treatment to form a coating film on the entire surface thereof. As shown in Fig. 2, the pushing member 6 is formed to have an almost cylindrical rod shape, and a male threaded section 21 for screwing into the female threading section 15 formed in the connection member 4 is formed on the outer peripheral surface of the pushing member. A star-shaped receded concave section 22 is formed in the upper surface of the pushing member 6 in the direction of screwing the pushing member 6 so as to insert a star-shaped wrench (not shown in the figure) for rotating the pushing member 6, in the same manner as the concave section 9 of the head section 8.

A method for the manufacture of the screw member 3, connection member 4, head section holding member 5, and pushing member 6 in the vertebra connection member 1 will be explained below. First, the screw member 3, connection member 4, head section holding member 5, and pushing member 6 are formed from titanium. Then, the screw member 3, connection member 4, head section holding member 5, and pushing member 6 are subjected to anodization treatment to form a coating film on the entire surface thereof. Then, the head section 8 of the screw member 3, the head support section 13 of the connection member 4, and the head holding section 16 of the head section holding member 5 are subjected to sandblasting treatment. The sandblasting treatment may be conducted on each of the head section 8 of the screw member 3, the head support section 13 of the connection member 4, and the head holding section 16 of the head section holding member 5, or on any one of them, or on any two of them. The screw member 3, connection member 4, head section holding member 5, and pushing member 6 can thus be manufactured.

A method for assembling the vertebra connection member 1 and a vertebra connection method will be described below.

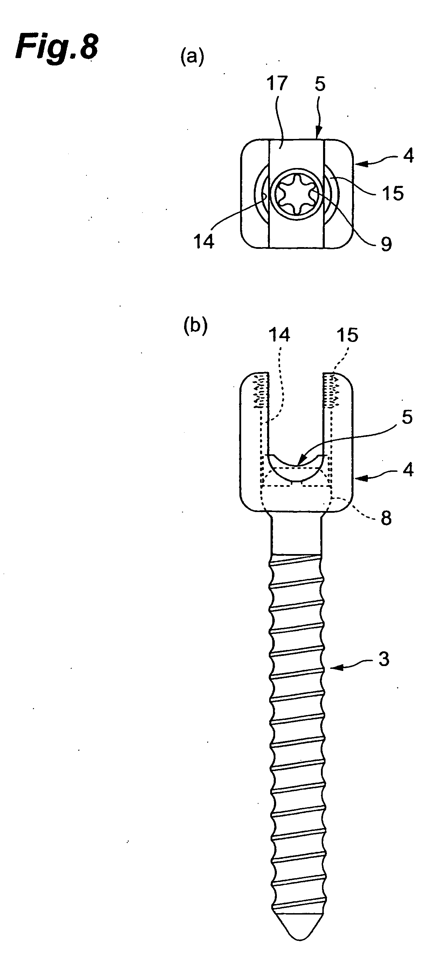

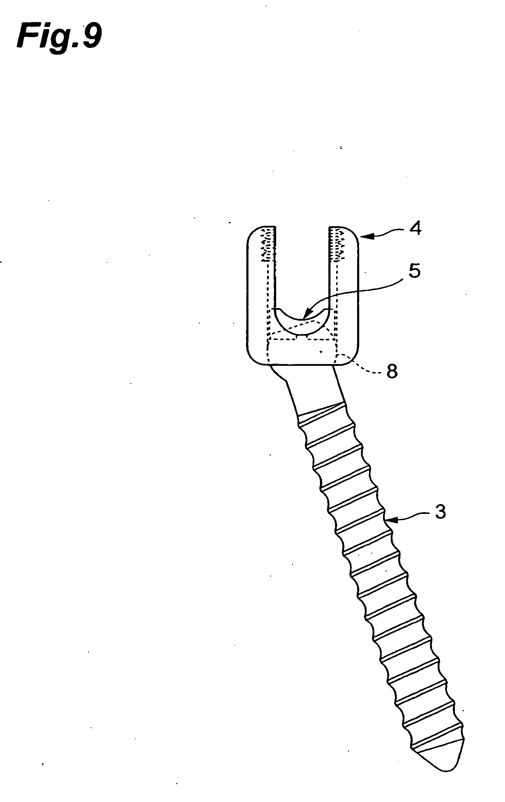

First, the threaded section 7 of the screw member 3 is inserted into the head support section 13 of the connection member 4, and the head section 8 is supported against the head support section 13. Then, the head section holding member 5 is inserted from the rod insertion section 24 of the connection member 4, the direction of the head section holding member 5 is set by the positioning section 12, the cylindrical section 18 is fitted into the port 23, and the head section 8 is held by the head holding section 16. As a result, as shown in Fig. 8 and Fig. 9, the screw member 3, connection member 4, head section holding member 5 are integrated, and the screw member 3 is held so that it can be tilted against the connection member 4.

The screw members 3 are then screwed into a plurality of vertebrae that have to be connected, so that the rod holding sections 17 of vertebra connection members 1 are disposed on a straight line. This screwing is performed by inserting the star-shaped wrench into the concave section 9 formed in the head section 8 and rotating the screw member 3.

Then, one rod 2 is inserted in each rod holding section 17, while correcting the tilting degree of the connection members 4, and the pushing member 6 is screwed into each connection member 4. As a result, the rod 2 and screw member 3 are connected and fixed to the connection member 4. A plurality of vertebrae are then connected and fixed by connecting and fixing a plurality of vertebra connection members 1 to the rod 2.

By sandblasting the surface of at least one section of the head section 8, head support section 13, and head holding section 16 in the vertebra connection member 1 as described hereinabove, the friction resistance of the head section 8 and the head support section 13 and/or head holding section 16 is greatly increased. As a result, with the vertebra connection member 1, by pushing the head section 8 toward the head support section 13 by the pushing member 6 via the rod 2 and head holding member 5, the head section 8 can be strongly fixed to the connection member 4 when the screw member 3 and rod 2 are connected. As a result, the displacement of the head section 8 with respect to the connection member 4 can be inhibited and the vertebrae can be reliably connected.

Here, surface roughening can be easily performed by sandblasting the surface of at least one section of the head section 8, head support section 13, and head holding section 16.

Furthermore, in the vertebra connection member 1, if the head section 8 is pushed by the pushing member 6 toward the head support section 13 via the rod 2 and head section support member 5, the head section 8 of the screw member 3 is inserted into the cylindrical section 24 of the head section holding member 5. As a result, the head section holding member 5 is elastically deformed so as to expand, and the restoration force of the head section holding member 5 acts to squeeze the head section 8. As a result, the head section holding member 5 and head section 8 can be stronger fixed to each other.

Furthermore, in the vertebra connection member 1, if the head section 8 is pushed by the pushing member 6 toward the head support section 13 via the rod 2 and head section support member 5, the notch 20 of the head section support member 5 will be impressed into the head section 8 of the screw member 3. As a result, with the vertebra connection member 1, the head section holding member 5 and head section 8 can be stronger fixed to each other.

Moreover, with the vertebra connection member 1, by forming the head support section 13 and head holding section 16 so that they have a concave curve surface that conforms to the head section 8, the contact surface area of the head section 8, head support section 13, and head holding section 16 is increased, whereby the head section 8 can be stronger fixed to the connection member 4.

Furthermore, with the vertebra connection member 1, by screwing the pushing member 6 on the inside of the connection member 4, the external dimensions of the vertebra connection member 1 can be reduced and the load applied to the tissue surrounding the vertebrae connected by the vertebra connection member 1 can be decreased.

In the present embodiment, the vertebra connection member 1 was explained in which the head section 8, head support section 13, and head holding section 16 were subjected to sandblasting, but the vertebra connection member may be also subjected to other surface roughening treatment instead of the sandblasting treatment or together therewith. For example, the surface roughening treatment may be conducted by chemical etching and sputtering.

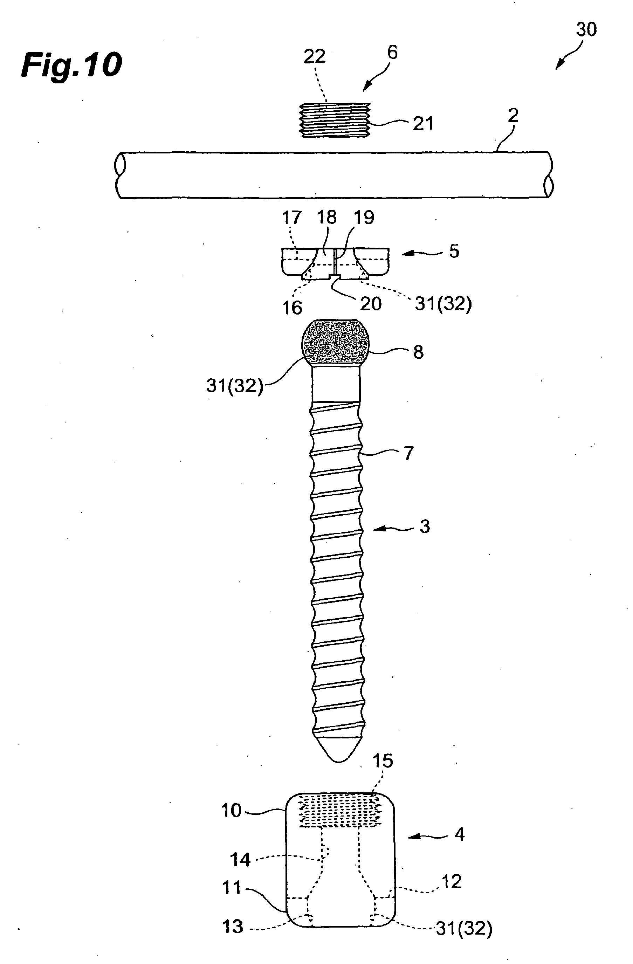

Furthermore, a slipping preventing agent 31 or adhesive 32 may be applied to at least one section of the head section 8, head support section 13, and head holding section 16 as in the vertebra connection member 30 shown in Fig. 10. In the vertebra connection member 30, slipping between the head section 8 and the head support section 13 and/or head holding section 16 is inhibited. As a result, by pushing the head section 8 by the pushing member 6 toward the head support section 13 via the rod 2 and head section support member 5, the head section 8 can be strongly fixed to the connection member 4 when the screw member 3 and rod 2 are connected. As a result, the displacement of the head section 8 with respect to the connection member 4 can be inhibited and the vertebrae can be reliably connected.

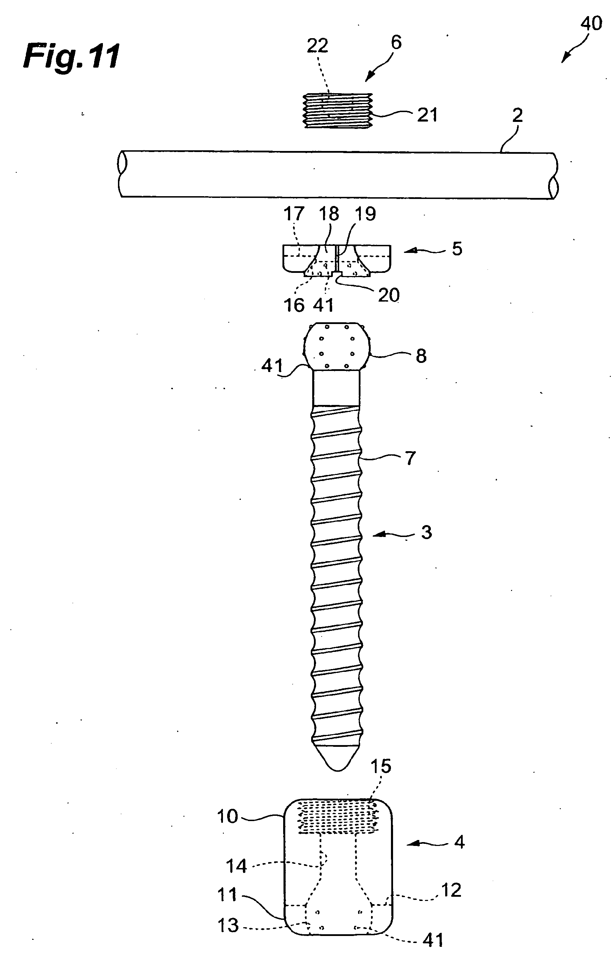

Furthermore, fine protrusions 41 may be provided on at least one section of the head section 8, head support section 13, and head holding section 16 as in the vertebra connection member 40 shown in Fig. 11. In the vertebra connection member 40, if the head section 8 is pushed by the pushing member 6 toward the head support section 13 via the rod 2 and head section support member 5, the protrusions 41 are impressed into the head section 8, head support section 13, and/or head holding section 16. As a result, the head section 8 can be more reliably fixed to the connection member 4 when the screw member 3 and rod 2 are connected.

It is clear from the explanation of the present invention above that numerous variations may be made in the present invention.

## Claims

1. A vertebra connection member (1) that connects a plurality of vertebrae via a rod (2), comprising:
a screw member (3) having a spherical head section (8) provided at one end of the screw member (3) and a screw section for being screwed into the vertebra;
a connection member (4) into which the screw member (3) is inserted and that connects the screw member (3) and the rod (2), said connection member (4) having a head support section (13) receiving the head section (8) and supporting the screw member (3) so that the screw member (3) can be tilted, said connection (4) member further having a rod insertion section (24) into which the rod (2) may be inserted;
a head section holding member (5) that is inserted between the rod (2) and the head section (8) inside the connection member (4), said head section holding member (5) having a head holding section (16) for holding the head section (8) and a rod holding section (17), inserted into the rod insertion section (24), for holding the rod (2); and
a pushing member (6) that pushes the head section (8) toward the head support section (13) via the rod (2) and the head section holding member (5),
**characterized in that**
at least one of the head section (8), the head support section (13), and the head holding section (16) is subjected to surface roughening treatment; and **in that**
the head section holding member (5) has an annular section (18) having the head holding section (16) on the lower inner wall surface and a slit (19) provided from the lower end to the upper end.

2. The vertebra connection member (1) according to claim 1, wherein the head section holding member (5) is provided with a notch (20) in the lower end portion of the annular section (18).

3. The vertebra connection member (1) according to claim 1, wherein the head support section (13) and the head holding section (16) are formed to have a concave curved surface shape that conforms to the head section (8).

4. The vertebra connection member (1) according to claim 1, wherein at least one section from among the head section (8), the head support section (13), and the head holding section (16) is provided with a protrusion (41).

5. The vertebra connection member (1) according to claim 1, wherein the pushing member (6) is screwed on the inner side of the connection member (4).

6. A vertebra connection member (1) that connects a plurality of vertebrae via a rod (2), comprising:
a screw member (3) having a spherical head section (8) provided at one end of the screw member (3) and a screw section for being screwed into the vertebra;
a connection member (4) into which the screw member (3) is inserted and that connects the screw member (3) and the rod (2), said connection member (4) having a head support section (13) receiving the head section (8) and supporting the screw member (3) so that the screw member (3) can be tilted, said connection member further having a rod insertion section (24) into which the rod (2) may be inserted;
a head section holding member (5) that is inserted between the rod (2) and the head section (8) inside the connection member (4), said head section holding member (5) having a head holding section (16) for holding the head section (8) and a rod holding section (17), inserted into the rod insertion section (24), for holding the rod (2); and
a pushing member (6) that pushes the head section (8) toward the head support section (13) via the rod (2) and the head section holding member (5),
**characterized in that**
a slipping preventing agent or an adhesive is applied to at least one of a surface between the head section (8) and the head support section (13) and a surface between the head section (8) and the head holding section (16); and **in that**
the head section holding member (5) has an annular section (18) having the head holding section (16) on the lower inner wall surface and a slit (19) provided from the lower end to the upper end.

7. The vertebra connection member (1) according to claim 6, wherein the head section holding member (5) is provided with a notch (20) in the lower end portion of the annular section (18).

8. The vertebra connection member (1) according to claim 6, wherein the head support section (13) and the head holding section (16) are formed to have a concave curved surface shape that conforms to the head section (18).

9. The vertebra connection member (1) according to claim 6, wherein at least one section from among the head section (8), the head support section (13), and the head holding section (16) is provided with a protrusion (41).

10. The vertebra connection member (1) according to claim 6, wherein the pushing member (6) is screwed on the inner side of the connection member (4).

## Patentansprüche

1. Wirbelverbindungselement (1), das mehrere Wirbel über eine Stange (2) verbindet, umfassend:
ein Schraubenelement (3), das einen sphärischen Kopfabschnitt (8), der an einem Ende des Schraubenelementes (3) bereitgestellt ist, und einen Schraubenabschnitt zum Einschrauben in den Wirbel aufweist;
ein Verbindungselement (4), in das das Schraubenelement (3) eingesetzt ist und das das Schraubenelement (3) und die Stange (2) verbindet, wobei das Verbindungselement (4) einen Kopflagerabschnitt (13) aufweist, der den Kopfabschnitt (8) aufnimmt und das Schraubenelement (3) so lagert, dass das Schraubenelement (3) geschwenkt werden kann, wobei das Verbindungselement (4) ferner einen Stangeneinsatzabschnitt (24) aufweist, in den die Stange (2) eingesetzt werden kann;
ein Kopfabschnittshalteelement (5), das zwischen die Stange (2) und den Kopfabschnitt (8) innerhalb des Verbindungselements (4) eingesetzt ist, wobei das Kopfabschnittshalteelement (5) einen Kopfhalteabschnitt (16) zum Halten des Kopfabschnitts (8) und einen Stangenhalteabschnitt (17) aufweist, eingesetzt in den Stangeneinsatzabschnitt (24), zum Halten der Stange (2); und
ein Drückelement (6), das den Kopfabschnitt (8) über die Stange (2) und das Kopfabschnittshalteelement (5) auf den Kopflagerabschnitt (13) zu drückt,
**dadurch gekennzeichnet, dass**
mindestens einer des Kopfabschnitts (8), des Kopflagerabschnitts (13) und des Kopfhalteabschnitts (16) einer Oberflächenaufraubehandlung unterzogen wird; und **dadurch**, dass
das Kopfabschnittshalteelement (5) einen ringförmigen Abschnitt (18) aufweist, der den Kopfhalteabschnitt (16) auf der unteren inneren Wandfläche und einen Schlitz (19) aufweist, der vom unteren zum oberen Ende bereitgestellt ist.

2. Wirbelverbindungselement (1) nach Anspruch 1, wobei das Kopfabschnittshalteelement (5) mit einer Nut (20) im unteren Endabschnitt des ringförmigen Abschnitts (18) versehen ist.

3. Wirbelverbindungselement (1) nach Anspruch 1, wobei der Kopflagerabschnitt (13) und der Kopfhalteabschnitt (16) derart ausgebildet sind, dass sie eine konkav gekrümmte Oberflächengestalt aufweisen, die dem Kopfabschnitt (8) entspricht.

4. Wirbelverbindungselement (1) nach Anspruch 1, wobei mindestens ein Abschnitt unter dem Kopfabschnitt (8), dem Kopflagerabschnitt (13) und dem Kopfhalteabschnitt (16) mit einem Vorsprung (41) versehen ist.

5. Wirbelverbindungselement (1) nach Anspruch 1, wobei das Drückelement (6) auf der Innenseite des Verbindungselements (4) verschraubt ist.

6. Wirbelverbindungselement (1), das mehrere Wirbel über eine Stange (2) verbindet, umfassend:
ein Schraubenelement (3), das einen sphärischen Kopfabschnitt (8), der an einem Ende des Schraubenelementes (3) bereitgestellt ist, und einen Schraubenabschnitt zum Einschrauben in den Wirbel aufweist;
ein Verbindungselement (4), in das das Schraubenelement (3) eingesetzt ist und das das Schraubenelement (3) und die Stange (2) verbindet, wobei das Verbindungselement (4) einen Kopflagerabschnitt (13) aufweist, der den Kopfabschnitt (8) aufnimmt und das Schraubenelement (3) so lagert, dass das Schraubenelement (3) geschwenkt werden kann, wobei das Verbindungselement ferner einen Stangeneinsatzabschnitt (24) aufweist, in den die Stange (2) eingesetzt werden kann;
ein Kopfabschnittshalteelement (5), das zwischen die Stange (2) und den Kopfabschnitt (8) innerhalb des Verbindungselements (4) eingesetzt ist, wobei das Kopfabschnittshalteelement (5) einen Kopfhalteabschnitt (16) zum Halten des Kopfabschnitts (8) und einen Stangenhalteabschnitt (17) aufweist, eingesetzt in den Stangeneinsatzabschnitt (24), zum Halten der Stange (2); und
ein Drückelement (6), das den Kopfabschnitt (8) über die Stange (2) und das Kopfabschnittshalteelement (5) auf den Kopflagerabschnitt (13) zu drückt,
**dadurch gekennzeichnet, dass**
ein Gleitverhinderungsmittel oder ein Klebstoff auf mindestens eine einer Oberfläche zwischen dem Kopfabschnitt (8) und dem Kopflagerabschnitt (13) und einer Oberfläche zwischen dem Kopfabschnitt (8) und dem Kopfhalteabschnitt (16) aufgebracht ist; und **dadurch**, dass
das Kopfabschnittshalteelement (5) einen ringförmigen Abschnitt (18) aufweist, der den Kopfhalteabschnitt (16) auf der unteren inneren Wandfläche und einen Schlitz (19) aufweist, der vom unteren zum oberen Ende bereitgestellt ist.

7. Wirbelverbindungselement (1) nach Anspruch 6, wobei das Kopfabschnittshalteelement (5) mit einer Nut (20) im unteren Endabschnitt des ringförmigen Abschnitts (18) versehen ist.

8. Wirbelverbindungselement (1) nach Anspruch 6, wobei der Kopflagerabschnitt (13) und der Kopfhalteabschnitt (16) derart ausgebildet sind, dass sie eine konkav gekrümmte Oberflächengestalt aufweisen, die dem Kopfabschnitt (8) entspricht.

9. Wirbelverbindungselement (1) nach Anspruch 6, wobei mindestens ein Abschnitt unter dem Kopfabschnitt (8), dem Kopflagerabschnitt (13) und dem Kopfhalteabschnitt (16) mit einem Vorsprung (41) versehen ist.

10. Wirbelverbindungselement (1) nach Anspruch 6, wobei das Drückelement (6) auf der Innenseite des Verbindungselements (4) verschraubt ist.

## Revendications

1. Membre de connexion vertébrale (1) qui connecte une pluralité de vertèbres par l'intermédiaire d'une tige (2), comprenant :
un membre de vis (3) ayant une section de tête sphérique (8) fournie à une extrémité du membre de vis (3) et une section de vis pour être vissée dans la vertèbre ;
un membre de connexion (4) dans lequel le membre de vis (3) est inséré et qui connecte le membre de vis (3) et la tige (2), ledit membre de connexion (4) ayant une section de support de tête (13)
recevant la section de tête (8) et supportant le membre de vis (3) de sorte que le membre de vis (3) puisse être incliné, ledit membre de connexion (4)
ayant en outre une section d'insertion de tige (24) dans laquelle la tige (2) peut être insérée ;
un membre de maintien de section de tête (5) qui est inséré entre la tige (2) et la section de tête (8) à l'intérieur du membre de connexion (4),
ledit membre de maintien de section de tête (5)
ayant une section de maintien de tête (16) pour maintenir la section de tête (8) et une section de maintien de tige (17), insérée dans la section d'insertion de tige (24), pour maintenir la tige (2) ; et
un membre pousseur (6) qui pousse la section de tête (8) vers la section de support de tête (13)
par l'intermédiaire de la tige (2) et le membre de maintien de section de tête (5),
**caractérisé en ce que**
au moins l'une de la section de tête (8), la section de support de tête (13), et la section de maintien de tête (16) est soumise à un traitement de rugosification de surface ; et **en ce que**
le membre de maintien de section de tête (5) a une section annulaire (18) ayant la section de maintien de tête (16) sur la surface de paroi intérieure inférieure et une fente (19) fournie de l'extrémité inférieure à l'extrémité supérieure.

2. Membre de connexion vertébrale (1) selon la revendication 1, dans lequel le membre de maintien de section de tête (5) est muni d'une encoche (20) dans la partie d'extrémité inférieure de la section annulaire (18).

3. Membre de connexion vertébrale (1) selon la revendication 1, dans lequel la section de support de tête (13) et la section de maintien de tête (16) sont formées pour avoir une forme de surface incurvée concave qui se conforme à la section de tête (8).

4. Membre de connexion vertébrale (1) selon la revendication 1, dans lequel au moins une section parmi la section de tête (8), la section de support de tête (13), et la section de maintien de tête (16) est munie d'une protubérance (41).

5. Membre de connexion vertébrale (1) selon la revendication 1, dans lequel le membre pousseur (6) est vissé sur le côté intérieur du membre de connexion (4).

6. Membre de connexion vertébrale (1) qui connecte une pluralité de vertèbres par l'intermédiaire d'une tige (2), comprenant :
un membre de vis (3) ayant une section de tête sphérique (8) fournie à une extrémité du membre de vis (3) et une section de vis pour être vissée dans la vertèbre ;
un membre de connexion (4) dans lequel le membre de vis (3) est inséré et qui connecte le membre de vis (3) et la tige (2), ledit membre de connexion (4) ayant une section de support de tête (13)
recevant la section de tête (8) et supportant le membre de vis (3) de sorte que le membre de vis (3) puisse être incliné, ledit membre de connexion (4)
ayant en outre une section d'insertion de tige (24) dans laquelle la tige (2) peut être insérée ;
un membre de maintien de section de tête (5) qui est inséré entre la tige (2) et la section de tête (8) à l'intérieur du membre de connexion (4),
ledit membre de maintien de section de tête (5)
ayant une section de maintien de tête (16) pour maintenir la section de tête (8) et une section de maintien de tige (17), insérée dans la section d'insertion de tige (24), pour maintenir la tige (2) ; et
un membre pousseur (6) qui pousse la section de tête (8) vers la section de support de tête (13) par l'intermédiaire de la tige (2) et le membre de maintien de section de tête (5),
**caractérisé en ce que**
un agent anti-glissement ou un adhésif est appliqué sur au moins l'une d'une surface entre la section de tête (8) et la section de support de tête (13) et une surface entre la section de tête (8) et la section de maintien de tête (16) ; et **en ce que**
le membre de maintien de section de tête (5) a une section annulaire (18) ayant la section de maintien de tête (16) sur la surface de paroi intérieure inférieure et une fente (19) fournie de l'extrémité inférieure à l'extrémité supérieure.

7. Membre de connexion vertébrale (1) selon la revendication 6, dans lequel le membre de maintien de section de tête (5) est muni d'une encoche (20) dans la partie d'extrémité inférieure de la section annulaire (18).

8. Membre de connexion vertébrale (1) selon la revendication 6, dans lequel la section de support de tête (13) et la section de maintien de tête (16) sont formées pour avoir une forme de surface incurvée concave qui se conforme à la section de tête (8).

9. Membre de connexion vertébrale (1) selon la revendication 6, dans lequel au moins une section parmi la section de tête (8), la section de support de tête (13), et la section de maintien de tête (16) est munie d'une protubérance (41).

10. Membre de connexion vertébrale (1) selon la revendication 6, dans lequel le membre pousseur (6) est vissé sur le côté intérieur du membre de connexion (4).
